# EUROPEAN PATENT SPECIFICATION

(11) **EP 4 253 854 B1**
(45) Date of publication and mention of the grant of the patent: **09.12.2026**
(21) Application number: 23165361.9
(22) Date of filing: 30.03.2023
(51) Int. Cl.: F24F 8/22, F24F 13/08, A61L 2/10

(54) **A STERILIZATION UNIT**
STERILISATIONSEINHEIT
UNITÉ DE STÉRILISATION

(30) Priority: 01.04.2022 TR 202205116
(43) Date of publication of application: 04.10.2023
(73) Proprietor: Arçelik Anonim Sirketi, 34445 Istanbul (TR)
(72) Inventor: ILGUN, Metin Mert, 34445 Istanbul (TR); HATIPOGLU, Ozcan, 34445 Istanbul (TR); KASAP, Fatih, 34445 Istanbul (TR); FILIZ, Deniz, 34445 Istanbul (TR); MEMON, Suleman Aijaz, 34445 Istanbul (TR)

(56) References cited:
- EP-A1- 1 541 179
- WO-A1-2022/046670
- US-A1- 2003 217 561

## Description

The present invention relates to a sterilization unit which is mounted onto an air conditioner indoor unit and which provides the sterilization of the ambient air.

Air conditioners are structures which change the indoor air as hot or cold, and generally comprise an indoor unit which provides heat extraction from the indoor environment and an outdoor unit which allows the extracted heat to be discharged to the outdoor environment. Today, with the increase of pathogen-borne diseases such as bacteria and viruses, the need for sterilization of the ambient air in addition to heating and cooling is increasing. Accordingly, manufacturers develop air conditioners comprising UVC lamps which eliminate dust, bacteria and viruses in the environment while providing heat exchange in the room. In the state of the art air conditioners with UVC lamps, the sterilization of the air drawn from the environment cannot be achieved sufficiently and the pathogens that cannot be eliminated spread further into the environment via the air flow generated. Systems using double UVC lamps to eliminate more pathogens increase electricity consumption as well as production cost. Moreover, the models with UVC lamps in the indoor unit of the air conditioner lead to an extra cost for the users as they cannot be adapted to the air conditioners used due to design constraints.

In the state of the art PCT Application No. WO2013155787A1, an air conditioner unit comprising a UV lamp is disclosed.

In the state of the art Chinese Application No. CN107883482A, an air conditioner unit comprising two opposite UV lamps is disclosed.

In the state of the art PCT Application No. WO2022046670A1, an excimer bulb assembly including an excimer bulb and a pass filter is disclosed.

In the state of the art European Application No. EP1541179A1, a sterilizing assembly to sterilize air in an air conditioning or recirculating system is disclosed.

In the state of the art United States Application No. US2003217561A, an air conditioner having sterilization and deodorization means is disclosed.

The aim of the present invention is the realization of a sterilization unit which is disposed onto an air conditioner indoor unit and which enables the ambient air to be efficiently sterilized before being received into the air conditioner indoor unit.

Another aim of the present invention is the realization of a sterilization unit compatible with the existing air conditioners.

The sterilization unit realized in order to attain the aim of the present invention, explicated in the first claim and the respective claims thereof, is suitable to be used with an air conditioner having an outdoor unit and an indoor unit whereon an air inlet for receiving the ambient air and an air outlet for discharging the ambient air with the temperate thereof being changed are arranged so as to be disposed onto the air inlet of the air conditioner, the sterilization unit comprising a body whereon an inlet opening for receiving air and an outlet opening for discharging the air to the air inlet are arranged, a chamber which is provided in the body and through which the ambient air received via the inlet opening to be sterilized is passed, at least one lamp housing which is provided in the chamber, a UVC lamp which is fitted into the lamp housing, at least one first reflector which is disposed on the lamp housing, which has an almost semicircular shape so as to partially surround the UVC lamp and which reflects the rays emitted by the UVC lamp, and at least one second reflector which is disposed in the chamber, which is positioned opposite to the UVC lamp so as to reflect back the rays emitted by the UVC lamp and which is in the form of a rectangular plate. Thus, the rays emitted by a single UVC lamp are reflected back by means of the second reflector, increasing the intensity of the UVC rays. Consequently, the air drawn into the air conditioner indoor unit is enabled to be exposed to a larger amount of UVC rays while passing through the sterilization unit such that the amount of pathogens eliminated is increased and the air is efficiently sterilized.

In the present invention, there is an upper grill which is placed onto the inlet opening at the upper side of the body, and the upper grill comprises bent fins so as to prevent the rays emitted by the UVC lamp from leaving the body. The fins have an almost V shape and prevent the rays from leaving the body through the inlet opening. Thus, the UVC rays harmful to the users are prevented from spreading out in the environment.

In an embodiment of the present invention, the upper grill comprises at least one first upper half-grill and at least one second upper half-grill which are positioned one above the other. Thus, ease of production is provided for the upper grill and cost advantage is also ensured since only the grill with a problem would be required to be replaced in case of a problem.

In the present invention, there is a lower grill which is positioned on the outlet opening arranged at the body lower side facing the air inlet, and the lower grill comprises bent fins so as to have an almost V shape. By means of the bent shape of the fins, the rays are prevented from leaking through the outlet opening. Thus, the UVC rays harmful to the users are prevented from spreading out in the environment.

In an embodiment of the present invention, the lower grill comprises at least one first lower half-grill, and at least one second lower half-grill and at least one third lower half-grill whereon the first lower half-grill is placed. Thus, ease of production is provided for the lower grill and cost advantage is also ensured since only the grill with a problem would be required to be replaced in case of a problem.

In an embodiment of the present invention, on the lamp housing, there is a guiding member having at least two plates extending parallel to each other towards the second reflector. By means of the plate, the amount of rays emitted from the UVC lamp towards the upper grill and/or the lower grill is decreased. Thus, the UVC rays are enabled to remain in the chamber and the sterilization efficiency is improved.

In an embodiment of the present invention, on the lamp housing, there is a quartz glass so as to cover the UVC lamp. The quartz glass enables the rays emitted by the UVC lamp to focus. Thus, the rays emitted by the UVC lamp and passed through the quartz glass move perpendicular to the second reflector. Consequently, the amount of rays reaching the second reflector at a right angle and reflected back into the chamber is increased.

By means of the present invention, with a sterilization unit used by being placed onto the air inlet of the air conditioner indoor unit, the air sucked by the air conditioner indoor unit is enabled to be cleansed of pathogens at high efficiency.

The sterilization unit realized in order to attain the aim of the present invention is illustrated in the attached figures, where:

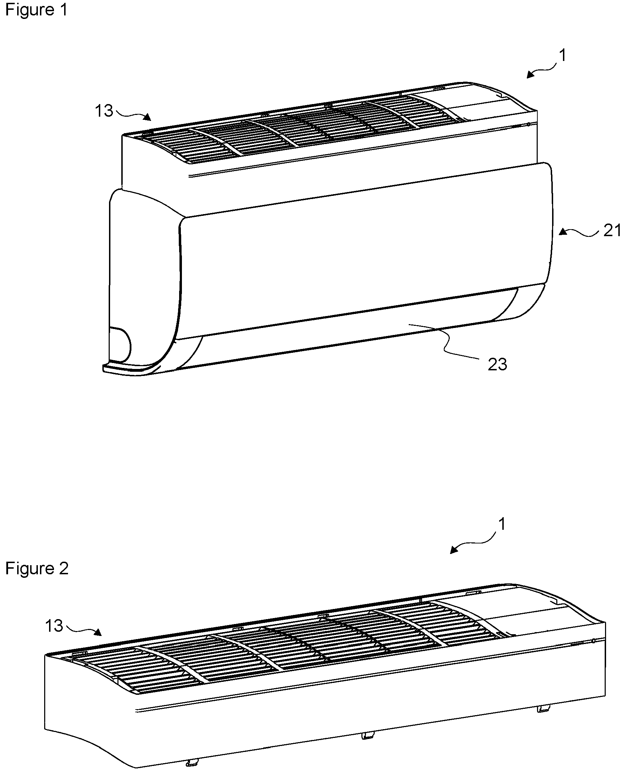

Figure 1 - is the perspective view of the sterilization unit related to an embodiment of the present invention on the air conditioner indoor unit.
Figure 2 - is the perspective view of the sterilization unit related to another embodiment of the present invention.

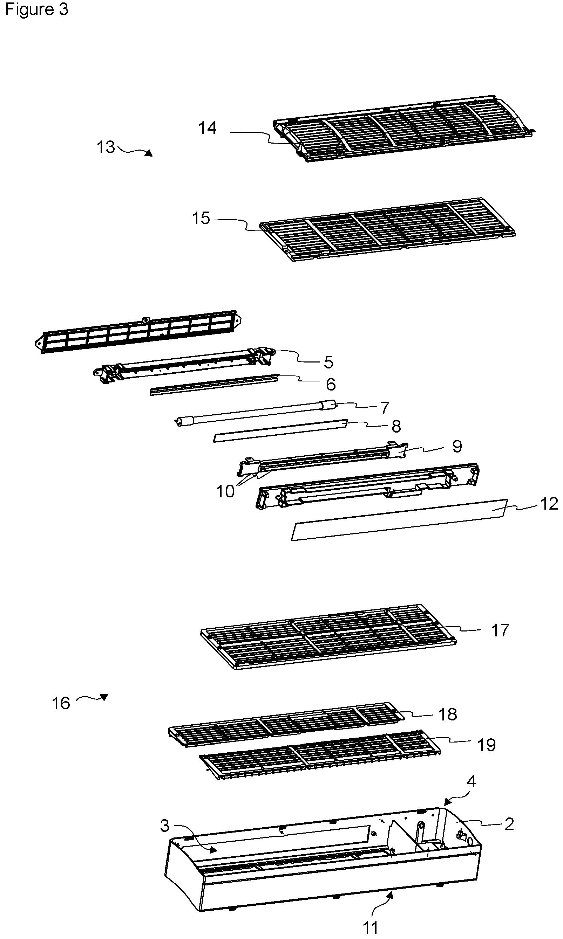

Figure 3 - is the exploded view of the sterilization unit related to another embodiment of the present invention.

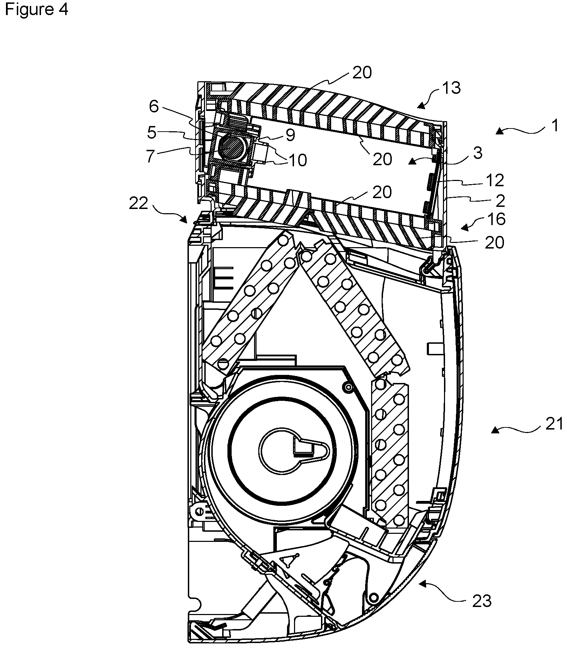

Figure 4 - is the sideways cross-sectional view of the sterilization unit and the air conditioner indoor unit related to an embodiment of the present invention.

The elements illustrated in the figures are numbered as follows:
1. Sterilization unit
2. Body
3. Chamber
4. Inlet opening
5. Lamp housing
6. First reflector
7. UVC lamp
8. Quartz glass
9. Guiding member
10. Plate
11. Outlet opening
12. Second reflector
13. Upper grill
14. First upper half-grill
15. Second upper half-grill
16. Lower grill
17. First lower half-grill
18. Second lower half-grill
19. Third lower grill
20. Fin
21. Air conditioner indoor unit
22. Air inlet
23. Air outlet

The sterilization unit (1) is suitable to be used with an air conditioner indoor unit (21) having an air inlet (22) for receiving the air so as to be placed onto the air inlet (22), and comprises a body (2), at least one inlet opening (4) which is arranged on the body (2), at least one outlet opening (11) which is arranged on the body (2) and which faces the air inlet (22), a chamber (3) which is provided in the body (2) and through which the air received via the inlet opening (4) is passed before being sent to the air inlet (22) through the outlet opening (11), at least one lamp housing (5) which is provided in the chamber (3), a UVC lamp (7) which is fitted into the lamp housing (5), and at least one first reflector (6) which is disposed on the lamp housing (5), which has a C shape so as to partially surround the UVC lamp (7) and which reflects the rays emitted by the UVC lamp (7). The sterilization unit (1) is suitable to be used by being placed on an indoor unit (21) of an air conditioner which has an air conditioner outdoor unit positioned outside and the air conditioner indoor unit (21) positioned inside. The air sucked by the air conditioner indoor unit (21) is received by the sterilization unit (1) which is placed onto the air inlet (22) without the need for a fan, and passes through the chamber wherein the same is subjected to UVC radiation, then reaching the inlet opening (4) of the air conditioner indoor unit (21).

The sterilization unit (1) of the present invention comprises at least one second reflector which is disposed in the chamber (3), which is positioned opposite to the UVC lamp so as to reflect back the rays emitted by the UVC lamp and which has a flat form. The second reflector (12) reflects back the rays emitted by the UVC lamp, thus increasing the intensity of the UVC rays in the chamber (3). The second reflector (12) has a flat form, preferably in the form of a rectangular plate. The second reflector (12) extends almost longitudinally in the chamber (3) so as to face the lamp housing (5). The rays emitted by the UVC lamp (7) hit the second reflector (12) so as to be reflected back to the UVC lamp (7) and the first reflector (6), thus increasing the intensity of rays in the chamber (3). By means of the second reflector (12) positioned opposite to the lamp housing (5), the rays emitted by the UVC lamp (7) are enabled to be reflected back into the chamber (3) and the efficiency of the air sterilization process is improved in a cost-effective manner by using only a single UVC lamp (7). Thus, the bacteria, virus and microbes in the air passing through the chamber (3) are subjected to more UVC rays, and the air is enabled to be efficiently purified.

In the present invention, the sterilization unit (1) comprises at least one upper grill (13) which is placed onto the inlet opening (4) and which has almost V-shaped fins (20). By means of the V-shape of the fins (20), the reflected rays are prevented from leaving the sterilization unit (1).

In an embodiment of the present invention, the upper grill (13) comprises at least one first upper half-grill (14) and at least one second upper half-grill (15) which is placed under the first upper half-grill (14). Half-fins extending at an angle with respect to each other are provided on the first upper half-grill (14) and the second upper half-grill (15). Thus, when the first upper half-grill (14) and the second upper half-grill (15) are placed one above the other, the half-fins align with each other, thus forming the V-shaped fins (20). By means of the two-piece structure of the upper grill, ease of production is provided.

In the present invention, the sterilization unit (1) comprises a lower grill (16) which is positioned on the body (2) lower side facing the air inlet (22) and which has almost V-shaped fins (20). The fins (20) on the lower grill (16) have a V-shaped cross-section, preventing the UVC rays from leaving the body (2).

In an embodiment of the present invention, the lower grill (16) comprises at least one first lower half-grill (17), and at least one second lower half-grill (18) and at least one third lower half-grill (19) which are placed under the first lower half-grill (17). The second lower half-grill (18) and the third lower half-grill (19) which are positioned as two pieces under the first lower half-grill (17) have half-fins extending at an angle with respect to each other so as to send air to the air inlet (22) at different angles. When the second lower half-grill (18) and the third lower half-grill (19) are placed under the first lower half-grill (16), the half-fins provided on the second lower half-grill (18) and the third lower half-grill (19) are aligned with the half-fins provided on the first lower half-grill 817), thus forming the V-shaped fins (20). Thus, the sterilized air is enabled to be homogeneously sent to the air inlet (22) of the air conditioner indoor unit (21).

In an embodiment of the present invention, the sterilization unit (1) comprises a guiding member (9) which is attached onto the lamp housing (5) and which has at least two plates (10) extending parallel to each other towards the second reflector (12). The plates (10) provided on the guiding member (9) enable the rays emitted by the UVC lamp (7) to be reflected towards the second reflector (12). Thus, the UVC rays are prevented from leaving the body (2).

In another embodiment of the present invention, on the lamp housing (5), there is a quartz glass (8) so as to cover the UVC lamp (7), and the quartz glass (8) enables the rays emitted by the UVC lamp (7) to be focused. Thus, the rays passing through the quartz glass (8) extend towards the second reflector (12) parallel to each other, by dispersing less. Consequently, since the rays are prevented from dispersing, the ray intensity in the chamber (3) is increased and fewer rays are refracted towards outside the body (2). Thereby, the sterilization efficiency in the chamber (3) is improved.

By means of the present invention, a sterilization unit (1) is realized, which enables the air sucked into the air conditioner indoor unit (21) to be cleansed of pathogens in a highly-efficient and cost-effective manner.

## Claims

1. A sterilization unit (1) suitable to be used with an air conditioner indoor unit (21) having an air inlet (22) for receiving the air so as to be placed onto the air inlet (22), and **comprising** a body (2), at least one inlet opening (4) which is arranged on the body (2), at least one outlet opening (11) which is arranged on the body (2) and which faces the air inlet (22), a chamber (3) which is provided in the body (2) and through which the air received via the inlet opening (4) is passed before being sent to the air inlet (22) through the outlet opening (11), at least one lamp housing (5) which is provided in the chamber (3), a UVC lamp (7) which is fitted into the lamp housing (5), and at least one first reflector (6) which is disposed on the lamp housing (5), which has a C shape so as to partially surround the UVC lamp (7) and which reflects the rays emitted by the UVC lamp (7), at least one second reflector which is disposed in the chamber (3), which is positioned opposite to the UVC lamp so as to reflect back the rays emitted by the UVC lamp and which has a flat form **characterized by** at least one upper grill (13) which is placed onto the inlet opening (4) and which has almost V-shaped fins (20) and at least one lower grill (16) which is placed onto the outlet opening (11) and which has almost V-shaped fins (20).

2. A sterilization unit (1) as in Claim 1, **characterized by** the upper grill (13) comprising at least one first upper half-grill (14) and at least one second upper half-grill (15) which is placed under the first upper half-grill (14).

3. A sterilization unit (1) as in Claim 1, **characterized by** the lower grill (16) comprising at least one first lower half-grill (17), and at least one second lower half-grill (18) and at least one third lower half-grill (19) which are placed under the first lower half-grill (17).

4. A sterilization unit (1) as in any one of the above claims, **characterized by** a guiding member (9) which is attached onto the lamp housing (5) and which has at least two plates (10) extending parallel to each other towards the second reflector (12).

5. A sterilization unit (1) as in any one of the above claims, **characterized by** a quartz glass (8) which is provided on the lamp housing (5) so as to cover the UVC lamp (7) and which enables the rays emitted by the UVC lamp (7) to be focused.

## Patentansprüche

1. Eine Sterilisationseinheit (1), die zur Verwendung mit einem Innenmodul (21) einer Klimaanlage geeignet ist, das über einen Lufteinlass (22) zur Aufnahme von Luft verfügt, sodass die Sterilisationseinheit auf den Lufteinlass (22) aufgesetzt werden kann, **umfasst** einen Körper (2), mindestens eine Einlassöffnung (4), die am Körper (2) angeordnet ist, mindestens eine Auslassöffnung (11), die am Körper (2) angeordnet ist und dem Lufteinlass (22) zugewandt ist, eine Kammer (3), die im Körper (2) vorgesehen ist und durch die die über die Einlassöffnung (4) aufgenommene Luft geleitet wird, bevor sie durch die Auslassöffnung (11) zum Lufteinlass (22) geleitet wird, mindestens ein Lampengehäuse (5), das in der Kammer (3) vorgesehen ist, eine UVC-Lampe (7), die in das Lampengehäuse (5) eingesetzt ist, und mindestens einen ersten Reflektor (6), der am Lampengehäuse (5) angeordnet ist, eine C-Form aufweist, um die UVC-Lampe (7) teilweise zu umschließen, und die von der UVC-Lampe (7) emittierten Strahlen reflektiert, mindestens einen zweiten Reflektor, der in der Kammer (3) angeordnet ist, der der UVC-Lampe gegenüberliegt, um die von der UVC-Lampe emittierten Strahlen zurückzuwerfen, und der eine flache Form aufweist, **gekennzeichnet ist sie dadurch**, dass mindestens ein oberes Rost (13), das auf die Einlassöffnung (4) aufgesetzt ist und fast V-förmige Lamellen (20) aufweist, sowie mindestens ein untere Rost (16), das auf die Auslassöffnung (11) aufgesetzt ist und fast V-förmige Lamellen (20) aufweist.

2. Eine Sterilisationseinheit (1), wie in Anspruch 1 aufgeführt, **ist dadurch gekennzeichnet, dass** der obere Rost (13) mindestens einen ersten oberen Halbrost (14) und mindestens einen zweiten oberen Halbrost (15) umfasst, der unter dem ersten oberen Halbrost (14) angeordnet ist.

3. Eine Sterilisationseinheit (1), wie in Anspruch 1 aufgeführt, **ist dadurch gekennzeichnet, dass** der untere Rost (16) mindestens eine erste untere Rostrahmenhälfte (17), mindestens eine zweite untere Rostrahmenhälfte (18) und mindestens eine dritte untere Rostrahmenhälfte (19) umfasst, die unterhalb der ersten unteren Rostrahmenhälfte (17) angeordnet sind.

4. Eine Sterilisationseinheit (1), wie in einem der vorherigen Ansprüchen aufgeführt, **ist dadurch gekennzeichnet, dass** ein am Lampengehäuse (5) befestigtes Führungselement (9) vorgesehen ist, dass mindestens zwei Platten (10) aufweist, die sich parallel zueinander in Richtung des zweiten Reflektors (12) erstrecken.

5. Eine Sterilisationseinheit (1), wie in einem der vorherigen Ansprüchen aufgeführt, **ist dadurch gekennzeichnet, dass** ein Quarzglas (8) am Lampengehäuse (5) vorgesehen ist, das die UVC-Lampe (7) abdeckt und die von der UVC-Lampe (7) emittierten Strahlen bündelt.

## Revendications

1. Unité de stérilisation (1) destinée à être utilisée avec une unité intérieure de climatiseur (21) comportant une entrée d'air (22) destinée à recevoir l'air, de manière à être placée sur l'entrée d'air (22), et **comprenant** un corps (2), au moins une ouverture d'entrée (4) qui est ménagée sur le corps (2), au moins une ouverture de sortie (11) qui est ménagée sur le corps (2) et qui fait face à l'entrée d'air (22), une chambre (3) qui est prévue dans le corps (2) et à travers laquelle passe l'air reçu par l'intermédiaire de l'ouverture d'entrée (4) avant d'être envoyé vers l'entrée d'air (22) par l'intermédiaire de l'ouverture de sortie (11), au moins un logement de lampe (5) qui est prévu dans la chambre (3), une lampe UVC (7) qui est montée dans le logement de lampe (5), et au moins un premier réflecteur (6) qui est disposé sur le logement de lampe (5), qui présente une forme en C de manière à entourer partiellement la lampe UVC (7) et qui réfléchit les rayons émis par la lampe UVC (7), au moins un deuxième réflecteur (12) qui est disposé dans la chambre (3), qui est positionné en regard de la lampe UVC (7) de manière à réfléchir les rayons émis par la lampe UVC (7) et qui présente une forme plate, **caractérisé par** au moins une grille supérieure (13) qui est placée sur l'ouverture d'entrée (4) et qui comporte des ailettes (20) présentant une forme sensiblement en V, et au moins une grille inférieure (16) qui est placée sur l'ouverture de sortie (11) et qui comporte des ailettes (20) présentant une forme sensiblement en V.

2. Unité de stérilisation (1) selon la revendication 1, **caractérisé par** la grille supérieure (13) comprenant au moins une première demi-grille supérieure (14) et au moins une deuxième demi-grille supérieure (15) qui est placée sous la première demi-grille supérieure.

3. Unité de stérilisation (1) selon la revendication 1, **caractérisé par** la grille inférieure (16) comprenant au moins une première demi-grille inférieure (17), au moins une deuxième demi-grille inférieure (18) et au moins une troisième demi-grille inférieure (19) qui sont placées sous la première demi-grille inférieure (17).

4. Unité de stérilisation (1) selon l'une quelconque des revendications précédentes, **caractérisé par** un élément de guidage (9) qui est fixé sur le logement de lampe (5) et qui comporte au moins deux plaques (10) s'étendant parallèlement l'une à l'autre vers le deuxième réflecteur (12).

5. Unité de stérilisation (1) selon l'une quelconque des revendications précédentes, **caractérisé par** un verre de quartz (8) qui est prévu sur le logement de lampe (5) de manière à recouvrir la lampe UVC (7) et qui permet de focaliser les rayons émis par la lampe UVC (7).
